# EUROPÄISCHE PATENTSCHRIFT

(11) **EP 1 078 911 B1**
(45) Veröffentlichungstag und Bekanntmachung des Hinweises auf die Patenterteilung: **17.11.2004**
(21) Anmeldenummer: 00114594.5
(22) Anmeldetag: 07.07.2000
(51) Int. Cl.: C07C 51/42, C07C 51/47, C07C 59/265

(54) **Verfahren zum Feinreinigen einer wässrigen Lösung, die eine fermentativ hergestellte organische Säure enthält**
Process for the fine purification of an aqueous solution containing an organic acid produced by fermentation
Procédé de purification fine d'une solution aqueuse contenant un acide organique obtenu par fermentation

(30) Priorität: 20.08.1999 DE 19939630
(43) Veröffentlichungstag der Anmeldung: 28.02.2001
(73) Patentinhaber: MG Technologies AG, 60325 Frankfurt am Main (DE)
(72) Erfinder: Boensch, Rudolf, Dr., 55299 Nackenheim (DE); Eichelsbacher, Michael, 55130 Mainz (DE); Hohmann, Klaus, 65719 Hofheim (DE); Pendl, Jiri, 32322 Pilzen (CZ); Hotek, Frantisek, 43985 Petrohrad (CZ); Cerny, Vaclav, 33151 Kaznejov (CZ)
(74) Vertreter: Revesz, Veronika

(56) Entgegenhaltungen:
- EP-A- 0 597 235
- EP-A- 0 913 385
- DE-A- 3 502 924
- US-A- 5 759 826

## Beschreibung

Die Erfindung betrifft ein Verfahren zum Feinreinigen einer wäßrigen Lösung, die eine fermentativ hergestellte Säure enthält, wobei die aus der Fermentation kommende Lösung zunächst vorgereinigt und in der Feinreinigung durch mindestens ein Adsorberharzbett geleitet wird, bevor man aus der wäßrigen Lösung Wasser mindestens teilweise entfernt.

Verfahren dieser Art sind aus dem US-Patent 5 759 826 und DE-A-197 47 902 bekannt.

Der Erfindung liegt die Aufgabe zugrunde, die bekannten Verfahren weiterzuentwickeln, um Kosten einzusparen und um insbesondere die Eindampfung der gereinigten Lösung zu vereinfachen und zu verbilligen.

Die Aufgabe wird beim eingangs genannten Verfahren dadurch gelöst, daß man die Lösung in der Feinreinigung durch das Adsorberharzbett leitet und dieses dabei mit der organischen Säure belädt, daß man das mit der organischen Säure beladene Adsorberharzbett mit Wasser oder einer wäßrigen anorganischen Säure eluiert und dabei ein wasserhaltiges Eluat erhält, welches man durch eine Umkehrosmose leitet, in welcher man mindestens 10 Gew.-% des Wassers abtrennt, welches im der Umkehrosmose zugeführten Eluat enthalten ist, und daß man die aus der Umkehrosmose kommende Lösung eindampft. Auf diese Weise lassen sich z. B. Citronensäure, Milchsäure, Bernsteinsäure, Weinsäure, Buttersäure und Propionsäure herstellen. Die Feinreinigung der wäßrigen Lösung dieser Säuren führt ohne weiteres zu den Säuren in Pharmaqualität.

Vorteilhafterweise verwendet man mindestens einen Teil des in der Umkehrosmose abgetrennten Wassers zum Eluieren des Adsorberharzbettes. Das in der Umkehrosmose abgetrennte Wasser enthält üblicherweise noch geringe Mengen der Produktsäure, so daß die Rückführung des Wassers zum Adsorberharzbett auch zur Rückgewinnung mindestens eines Teils dieser Säure führt. Beim Adsorberharzbett kann es sich z. B. auch um einen Anionenaustauscher handeln.

Das Adsorberharzbett, welches bei der Feinreinigung die Säure bindet, kann in einer Weiterbildung des Verfahrens als Elektrodialyse-Kammer ausgebildet werden. Hierbei kann es zweckmäßig sein, die der Kammer aufgegebene Lösung zum Verbessern der elektrischen Leitfähigkeit mit einer geringen Menge einer starken Säure, z. B. Schwefelsäure, zu versetzen. Um diese Säure dann aus dem Eluat wieder zu entfernen, kann ein weiteres Adsorberharzbett (z. B. ein Anionenaustauscher) vorteilhaft sein, durch den das Eluat vor der Umkehrosmose geführt wird. Dieses Adsorberharzbett ist im Anfangszustand mit der zu gewinnenden organischen Säure beladen.

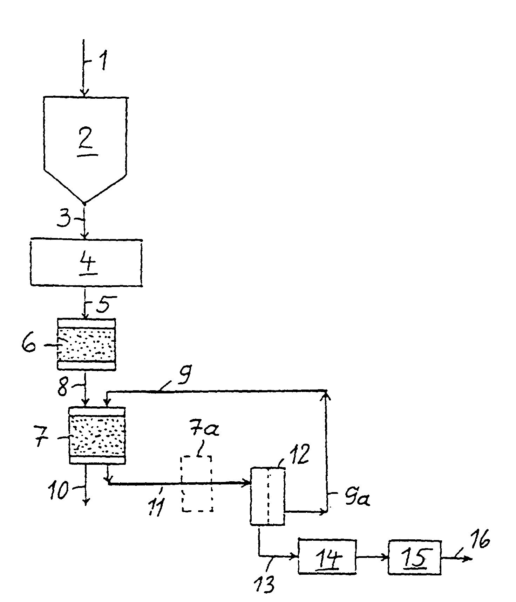

Ausgestaltungsmöglichkeiten des Verfahrens werden mit Hilfe der Zeichnung erläutert. Die Zeichnung zeigt ein Fließschema des Verfahrens.

Durch die Leitung (1) wird ein kohlehydrathaltiger Rohstoff einer Fermentation (2) zugeführt, welche an sich bekannt ist und üblicherweise aus mehreren parallelen Fermentern besteht. Man erhält eine erste säurehaltige Lösung, die man in der Leitung (3) abzieht und einer ebenfalls bekannten Vorreinigung (4) zuführt. Man arbeitet hierbei mit mehreren Filtrationsstufen und dabei auch üblicherweise mit einer Ultrafiltration sowie eventuell auch noch mit einer Entfärbung, z. B. mittels Aktivkohle. Man erhält eine vorgereinigte zweite wäßrige Lösung der Produktsäure, die man in der Leitung (5) abzieht. Bei der anschließenden Feinreinigung setzt man im vorliegenden Fall einen ersten Anionenaustauscher (6) und ein Adsorberharzbett (7) ein, bei dem es sich z. B. um einen zweiten Anionenaustauscher handeln kann.

Der erste Anionenaustauscher (6) ist im Anfangszustand mit der Produktsäure, z. B. Citronensäure, beladen und bindet vor allem Anionen, z. B. Sulfat- und Nitrationen. Nach dem Durchlauf durch den ersten Anionenaustauscher (6) führt man die wäßrige Lösung der Produktsäure durch die Leitung (8) in das Adsorberharzbett (7). Dieses Bett (7) ist anfangs mit OH⁻-Ionen beladen und bindet die Produktsäure. Der Ablauf aus dem Bett (7) enthält die abzutrennenden Verunreinigungen und wird in der Leitung (10) abgezogen und üblicherweise verworfen.

Wenn das Adsorberharzbett (7) mit der Produktsäure beladen ist, wird es regeneriert und zu diesem Zweck zunächst mit Wasser oder einer wäßrigen anorganischen Säure (z. B. Schwefelsäure) eluiert. Die Elutionsflüssigkeit wird durch die Leitung (9) herangeführt. Durch die Elution entsteht eine verdünnte wäßrige Lösung der Produktsäure, die man in der Leitung (11) abzieht und durch eine Umkehrosmose (12) führt. Die Umkehrosmose sorgt dafür, daß mindestens 10 Gew.-% des Wassers der in der Leitung (11) herangeführten Lösung abgetrennt werden und in der Leitung (9a) abgezogen werden können. Falls Wasser zum Eluieren verwendet wird, trennt man in der Umkehrosmose (12) vorzugsweise mindestens 50 Gew.-% des in der Leitung (11) herangeführten Wassers ab; falls man mit verdünnter Säure eluiert, trennt man in der Umkehrosmose (12) vorzugsweise mindestens 20 Gew.-% des in der Leitung (11) herangeführten Wassers ab. Dieses Wasser, das zumeist noch geringe Mengen der Produktsäure enthält, wird zweckmäßigerweise durch die Leitung (9a) zum Eluieren zurückgeführt.

Die aus der Umkehrosmose (12) ablaufende teilkonzentrierte Produktlösung gelangt in der Leitung (13) zunächst zu einer Eindampfung (14) und dann zu einer Kristallisation (15). Man erhält eine Produktsäure in der Leitung (16), die weitgehend wasserfrei ist und z. B. noch getrocknet wird, wobei ihre Reinheit die verschiedenen internationalen Pharmaspezifikationen erfüllt.

Wenn man das Adsorberharzbett (7) als Elektrodialyse-Kammer ausbildet, wobei zum Verbessern der elektrischen Leitfähigkeit der Lösung z. B. Schwefelsäure zugegeben wird, kann sich ein weiterer Anionenaustauscher (7a) empfehlen (gestrichelt angedeutet), durch den das Eluat der Leitung (11) geführt wird. Zweckmäßigerweise ist dieser Anionenaustauscher (7a) im Anfangszustand mit der Produktsäure beladen, so daß die Sulfationen gebunden werden können. Das so gereinigte Eluat führt man dann in die Umkehrosmose (12).

### Beispiel:

Man arbeitet mit einer der Zeichnung entsprechenden Anordnung, in welcher man fermentativ hergestellte Citronensäure feinreinigt. Die vorgereinigte Lösung, die aus einer Ultrafiltration kommt, wird in der Leitung (5) mit 200 g/l Citronensäure einem mit Citronensäure vorbeladenen Anionenaustauscher (6) (Lewatit von Bayer) zugeführt. Wenn die Beladekapazität des Austauschers (6) für Anionen erschöpft ist, wird er mit verdünnter Natronlauge regeneriert. Das Adsorberharzbett (7) ist mit Anionenaustauschermaterial (Lewatit) gefüllt und bindet Citronensäure. Nach der Beladung wird das Harz mit Wasser eluiert, wobei die anfallende wäßrige Lösung 10 g/l reine Citronensäure enthält. In der handelsüblichen Umkehrosmose (12) von UNION Filtration (Dänemark) fällt in der Leitung (13) eine wäßrige Lösung mit 170 g/l Citronensäure an, welche leicht eingedampft werden kann. Das Wasser der Leitung (9), welches noch 5 g/l Citronensäure enthält, wird zum Eluieren im Bett (7) zurückgeführt.

## Patentansprüche

1. Verfahren zum Feinreinigen einer wäßrigen Lösung, die eine fermentativ hergestellte organische Säure enthält, wobei die aus der Fermentation kommende Lösung zunächst vorgereinigt und in der Feinreinigung durch mindestens ein Adsorberharzbett geleitet wird, bevor man aus der wäßrigen Lösung Wasser mindestens teilweise entfernt, **dadurch gekennzeichnet, daß** man die Lösung in der Feinreinigung durch das Adsorberharzbett leitet und dieses dabei mit der organischen Säure belädt, daß man das mit der organischen Säure beladene Adsorberharzbett mit Wasser oder einer wäßrigen anorganischen Säure eluiert und dabei ein wasserhaltiges Eluat erhält, welches man durch eine Umkehrosmose leitet, in welcher man mindestens 10 Gew.-% des Wassers abtrennt, welches im der Umkehrosmose zugeführten Eluat enthalten ist, und daß man die aus der Umkehrosmose kommende Lösung eindampft.

2. Verfahren nach Anspruch 1, **dadurch gekennzeichnet, daß** man mindestens einen Teil des in der Umkehrosmose abgetrennten Wassers zum Eluieren des Adsorberharzbettes verwendet.

3. Verfahren nach Anspruch 1 oder 2, **dadurch gekennzeichnet, daß** das Adsorberharzbett ein Anionenaustauscher ist.

4. Verfahren nach Anspruch 1 oder 2, **dadurch gekennzeichnet, daß** das Adsorberharzbett als Elektrodialyse-Kammer ausgebildet ist.

5. Verfahren nach Anspruch 1 oder einem der folgenden, **dadurch gekennzeichnet, daß** man das Eluat zum Entfernen von Säureresten durch einen weiteren Anionenaustauscher leitet, der im Anfangszustand mit der organischen Säure beladen ist und welcher im Eluat befindliche Verunreinigungen bindet, und daß man das so gereinigte Eluat in die Umkehrosmose leitet.

## Claims

1. A process of fine cleaning an aqueous solution which contains an organic acid produced by fermentation, wherein the solution coming from the fermentation is first of all precleaned and in the fine cleaning stage is passed through at least one adsorber resin bed before water is at least partly removed from the aqueous solution, **characterised in that** in the fine cleaning stage the solution is passed through the adsorber resin bed and the same is loaded with the organic acid, that the adsorber resin bed loaded with the organic acid is eluted with water or an aqueous inorganic acid, whereby a water-containing eluate is obtained, which is passed through a reverse osmosis stage in which at least 10 wt-% of the water contained in the eluate supplied to the reverse osmosis stage is separated off, and that the solution coming from the reverse osmosis stage is evaporated.

2. A process according to claim 1, **characterised in that** at least part of the water separated in the reverse osmosis stage is used for eluting the adsorber resin bed.

3. A process according to claim 1 or 2, **characterised in that** the adsorber resin bed is an anion exchanger.

4. A process according to claim 1 or 2, **characterised in that** the adsorber resin bed is designed as an electrodialysis chamber.

5. A process according to claim 1 or any of the following claims, **characterised in that** to remove acid residues the eluate is passed through a further anion exchanger which in the initial state is loaded with the organic acid and binds impurities contained in the eluate, and that the eluate thus purified is introduced into the reverse osmosis stage.

## Revendications

1. Procédé de purification fine d'une solution aqueuse, qui contient un acide organique préparé par fermentation, la solution provenant de la fermentation étant d'abord pré-purifiée et passant dans la purification fine par au moins un lit de résine adsorbante avant que de l'eau soit éliminée au moins en partie de la solution aqueuse, **caractérisé en ce que** l'on fait passer la solution dans l'épuration fine dans le lit de résine adsorbante et on charge celui-ci ainsi de l'acide organique, **en ce que** l'on élue le lit de résine adsorbante, chargé de l'acide organique, par de l'eau ou par un acide minéral aqueux et on obtient ainsi un éluat contenant de l'eau que l'on fait passer dans une osmose inverse, dans laquelle on sépare au moins 10 % en poids de l'eau qui est contenue dans l'éluat envoyé à l'osmose inverse et **en ce que** l'on concentre par évaporation la solution arrivant de l'osmose inverse.

2. Procédé suivant la revendication 1, **caractérisé en ce que** l'on utilise au moins une partie de l'eau séparée dans l'osmose inverse pour éluer le lit de résine adsorbante.

3. Procédé suivant la revendication 1 ou 2, **caractérisé en ce que** le lit de résine absorbante est un échangeur d'anions.

4. Procédé suivant la revendication 1 ou 2, **caractérisé en ce que** le lit de résine adsorbante est constitué en chambre d'électrodialyse.

5. Procédé suivant la revendication 1 ou l'une des suivantes, **caractérisé en ce que** l'on fait passer l'éluat pour éliminer des restes d'acide dans un autre échangeur d'anions, qui est chargé à l'état initial de l'acide organique et qui fixe les impuretés se trouvant dans l'éluat et **en ce que** l'on envoie l'éluat ainsi purifié à l'osmose inverse.
